# EUROPÄISCHE PATENTSCHRIFT

(11) **EP 1 440 155 B1**
(45) Veröffentlichungstag und Bekanntmachung des Hinweises auf die Patenterteilung: **12.11.2008**
(21) Anmeldenummer: 02782936.5
(22) Anmeldetag: 17.10.2002
(51) Int. Cl.: C12N 15/55, C12N 9/20, C12N 5/10

(54) **LIPASE-VARIANTEN**
LIPASE VARIANTS
VARIANTES DE LIPASE

(30) Priorität: 22.10.2001 DE 10151292; 08.02.2002 DE 10205444
(43) Veröffentlichungstag der Anmeldung: 28.07.2004
(73) Patentinhaber: BASF SE, 67056 Ludwigshafen (DE)
(72) Erfinder: MATUSCHEK, Markus, 69469 Weinheim (DE); STÜRMER, Rainer, 67127 Rödersheim-Gronau (DE); HAUER, Bernhard, 67136 Fussgönheim (DE); KLEBE, Gerhard, 35039 Marburg (DE); BOCOLA, Marco, 42499 Hückeswagen (DE)
(86) Internationale Anmeldenummer: PCT/EP2002/011620
(87) Internationale Veröffentlichungsnummer: WO 2003/035878

(56) Entgegenhaltungen:
- EP-A- 0 548 228
- WO-A-95/35381
- WO-A1-00/32758
- SCHULZ, T.: "Molekulare Grundlagen der Stereoselektivität Lipase-katalysierter Umsetzungen" [Online] 19. März 2001 (2001-03-19), , STUTTGART , XP002346324 Gefunden im Internet: URL:http://opus.rus.uni-stuttgart.de/opus/ volltexte/2001/843/pdf/tanja_diss.pdf> [gefunden am 2005-09-21] Kapitel 5.8, 6.5, 6.6 * Tabellen 21-23 *
- BOCOLA, M.: "Strukturelle und kinetische Untersuchungen zum Mechanismus und Vorhersage der stereoselektiven Substraterkennung von Lipasen" [Online] 12. Februar 2002 (2002-02-12), , MARBURG , XP002346325 Gefunden im Internet: URL:http://archiv.ub.uni-marburg.de/diss/z 2002/0104/pdf/dmb.pdf> [gefunden am 2005-09-21] * Seite 127 - Seite 139 * * Seite 139, linke Spalte, Absatz 1 * Seite 127, Tabelle
- HWANG BUM-YEOL ET AL: "Computer-aided molecular modeling of the enantioselectivity of Pseudomonas cepacia lipase toward gamma- and delta-lactones" JOURNAL OF MOLECULAR CATALYSIS B ENZYMATIC, Bd. 10, Nr. 1-3, 4. September 2000 (2000-09-04), Seiten 223-231, XP002346313 ISSN: 1381-1177
- TUOMI W V ET AL: "Molecular basis for enantioselectivity of lipase from Pseudomonas cepacia toward primary alcohols. Modeling, kinetics, and chemical modification of Tyr29 to increase or decrease enantioselectivity" JOURNAL OF ORGANIC CHEMISTRY 16 APR 1999 UNITED STATES, Bd. 64, Nr. 8, 16. April 1999 (1999-04-16), Seiten 2638-2647, XP002346314 ISSN: 0022-3263
- GENTNER C ET AL: "Primary alcohols in a ring structure: Quantifying enantioselectivity of Pseudomonas cepacia lipase by an in silico assay" COLLOIDS AND SURFACES B: BIOINTERFACES 2002 NETHERLANDS, Bd. 26, Nr. 1-2, September 2002 (2002-09), Seiten 57-66, XP002346315 ISSN: 0927-7765
- NARDINI MARCO ET AL: "Crystal structure of Pseudomonas aeruginosa lipase in the open conformation: The prototype for family I.1 of bacterial lipases." JOURNAL OF BIOLOGICAL CHEMISTRY, Bd. 275, Nr. 40, 6. Oktober 2000 (2000-10-06), Seiten 31219-31225, XP002244975 ISSN: 0021-9258
- FRENKEN L G J ET AL: "CLONING OF THE PSEUDOMONAS FLUMAE LIPASE GENE AND DETERMINATION OF THE ACTIVE SITE RESIDUES" APPLIED AND ENVIRONMENTAL MICROBIOLOGY, WASHINGTON,DC, US, Bd. 58, Nr. 12, 1. Dezember 1992 (1992-12-01), Seiten 3787-3791, XP000569445 ISSN: 0099-2240
- SCHULZ TANJA ET AL: "Stereoselectivity of Pseudomonas cepacia lipase toward secondary alcohols: A quantitative model" PROTEIN SCIENCE, Bd. 9, Nr. 6, Juni 2000 (2000-06), Seiten 1053-1062, XP002346316 ISSN: 0961-8368
- KIM MYUNG HEE ET AL: "Substitution of glycine 275 by glutamate (G275E) in lipase of Bacillus Stearothermophilus affects its catalytic activity and enantio-and chain length specificity" JOURNAL OF MICROBIOLOGY AND BIOTECHNOLOGY, KOREAN SOCIETY FOR APPLIED MICROBIOLOGY, SEOUL, KO, Bd. 10, Nr. 5, 2000, Seiten 764-769, XP002902706 ISSN: 1017-7825
- LIEBETON K ET AL: "DIRECTED EVOLUTION OF AN ENANTIOSELECTIVE LIPASE" CHEMISTRY AND BIOLOGY, CURRENT BIOLOGY, LONDON, GB, Bd. 7, 2000, Seiten 709-718, XP001018063 ISSN: 1074-5521

## Beschreibung

Die vorliegende Erfindung betrifft neue Lipase-Varianten mit verbesserten Eigenschaften, Verfahren zur Herstellung dieser Lipase-Varianten, sowie die Verwendung dieser Lipase-Varianten als Katalysatoren in chemischen Reaktionen, insbesondere zur Herstellung von optisch aktiven Verbindungen.

Der Einsatz der natürlich vorkommenden Biokatalysatoren in chemischen Reaktionen wird durch zahlreiche Faktoren limitiert wie beispielsweise mangelnde Aktivität, Stabilität und Enantioselektivität. Daher ist es wünschenswert, neue Biokatalysatoren zur Verfügung zu haben, die hinsichtlich dieser Eigenschaften optimiert worden sind.

Eine industriell bedeutende Anwendung von Lipasen liegt in der Herstellung von optisch aktiven Aminen, Alkoholen, Carbonsäuren und Carbonsäureestern.

Aus EP 0 548 228 sind optimierte Lipase-Varianten für den Einsatz als Waschmitteladditive bekannt.

WO 95/35381 beschreibt Lipase Varianten aus Pseudomonas glumae.

Nardini Marco et al , J Biol Chemistry Bd. 275, Nr. 40, 6 Oktober 2000, Seiten 31219-31225, beschreiben die Kristallstruktur von Pseudomonas aeruginosa Lipase.

WO 00/32758 schlägt mögliche Aminosäuremodifikationen an bestimmten Positionen von Pseudomonas Lipasen vor

Der Erfindung lag die Aufgabe zu Grunde, neue Lipase-Varianten mit verbesserten Eigenschaften, zum Beispiel erhöhter spezifische Aktivität zur Verfügung zu stellen.

Gefunden wurden Lipase- Varianten, die herstellbar sind, indem man an der Aminosäuresequenz einer Ausgangslipase SEQ ID NO:1 eine Aminosäuresubstitution an Position 17. durchführt, wobei Leu 17 ersetzt wird durch Ala 17.

Ein weiterer Gegenstand der Erfindung sind Nukleinsäuresequenzen, die die vorstehend beschriebenen erfindungsgemäßen Lipase-Varianten kodieren. Geeignete Nukleinsäuresequenzen sind durch Rückübersetzung der Polypeptidsequenz gemäß dem genetischen Code erhältlich.

Bevorzugt werden dafür solche Codons verwendet, die entsprechend der Organismus spezifischen *codon usage* häufig verwendet werden. Die *codon usage* läßt sich anhand von Computerauswertungen anderer, bekannter Gene des betreffenden Organismus leicht ermitteln.

Soll die Lipase-Variante beispielsweise in einem Bakterium exprimiert werden, so ist es häufig vorteilhaft, die *codon usage* des Bakteriums bei der Rückübersetzung zu verwenden.

Ein weiterer Gegenstand der Erfindung sind Nukleinsäurekonstrukte, die die erfindungsgemäßen Nukleinsäuresequenzen enthalten. Diese Nukleinsäurekonstrukte tragen bevorzugt neben den Nukleinsäuresequenzen noch regulatorische Sequenzen die beispielsweise für die Expression, die Replikation oder die Rekombination vorteilhaft sind.

Ein weiterer Gegenstand der Erfindung sind Wirtsorganismen, die mit diesen Nukleinsäurekonstrukten transformiert werden können. Als Wirtsorganismen geeignet sind Ein- oder Mehrzeller, wobei Mikroorganismen als Wirtsorganismen bevorzugt sind.

Ein weiterer Gegenstand der Erfindung sind Verfahren zur Herstellung von Lipase-Varianten, indem man Wirtsorganismen, die mit einer für die Lipase-Varianten codierendenden Nukleinsäuresequenz transformiert wurden, unter solchen Bedingungen kultiviert, die die Produktion der Lipase-Varianten in diesen Wirtsorganismen erlauben. Anschließend, wenn die Konzentration der Lipase-Variante in dem Kulturmedium die gewünschte Größe erreicht hat, kann die Lipase-Variante aus dem Kulturmedium isoliert werden. Je nach gewünschtem Einsatzzweck der Lipase kann diese noch mit üblichen Verfahren der Proteinchemie wie Fällung und Chromatographieaufgereinigt werden. Für manche Einsatzzwecke kann jedoch auch der gesamte Wirtsorganismus ggf. nach vorheriger Abtötung verwendet werden.

Die Erfindung betrifft weiterhin Lipasen-Formulierungen, enthaltend mindestens eine der erfindungsgemäßen Lipase-Varianten.

Die Lipasen-Varianten können in diesen Formulierung in isolierter Form, auf einem festen Trägermaterial immobilisiert oder in Zellen verwendet werden. Solche Lipase Formulierungen können neben der Lipase-Variante auch noch Stabilisatoren, Detergentien sowie Enzymsubstrate enthalten.

Methoden zur Immobilisierung von Lipasen sind beispielsweise in WO 00/05354, EP 1069183 beschrieben.

Bevorzugt werden die Lipase-Varianten in immobilisierter Form verwendet.

Die Erfindung betrifft weiterhin ein Verfahren zur enzymkatalytischen Umwandlung oder enantioselektiven Umwandlung von Substraten, indem man die Substrate in Gegenwart der erfindungsgemäßen Lipase umsetzt.

Die erfindungsgemäßen Lipase-Varianten können demgemäß als Katalysatoren in chemischen Reaktionen verwendet werden. Die erfindungsgemäßen Lipase-Varianten sind in einer Vielzahl von chemischen Raktionen einsetzbar.

Die Messung der Katalytischen Eigenschaften der erfindungsgemäßen Lipase-Varianten erfolgt mit Referenzreaktionen. Damit lassen sich auch gut höhere spezifische Aktivitäten gegenüber den Ausgangslipasen ermitteln:
Die Bestimmung der spezifischen Aktivität einer Lipase-Variante erfolgt erfindungsgemäß beispielsweise in folgender Referenz-Reaktion, in der die Lipase oder Lipase-Variante die Umsetzung von racemischen trans-Methoxycyclohexanol mit Vinyllaurat zu Laurinsäure-1R,2R-2-Methoxycyclohexylester und 1S,2S-Methoxycyclohexanol katalysiert.

Die Bestimmung der spezifischen Aktivität erfolgt in dieser Referenzreaktion unter folgenden Bedingungen:
Die Referenzrektionreaktion wird in Mikrotiterplatten mit 96 Kavitäten durchgeführt. Die Mikrotiterplatte enthält pro Kavität
   A das Lipase-haltige Lyophyllisat von 150 µl Kulturüberstand von Kulturen natürlicher oder rekombinanter Organismen, die eine Lipase exprimieren oder
   B die entsprechende, fest vorgegebene Menge isolierte Lipase.

Für die Racemattrennung werden racemisches trans-Methoxycyclohexanol und Vinyllaurat im molaren Verhältnis 1 : 0,65 eingesetzt [d.h. pro Platte 10 g Methoxycyclohexanol und 11,3 g Vinyllaurat]. In jedes Well werden 200 µl dieses Gemisches pipettiert.

Jede Platte wird mit ,plate sealer' versiegelt, mit Deckel verschlossen und 96 h bei Raumtemperatur im Rundschüttler bei 150 U/min inkubiert. Anschließend werden jeweils 100 µl pro Kavität abgenommen mit Essigsäureethylester versetzt und gaschromatographisch analysiert.

Gemessen wird die Menge gebildetes Methoxycyclohexanyllaurat bezogen auf die Menge der eingesetzten Lipase (Fall B) oder bezogen auf die optische Dichte der Zellsuspensionen in den Kavitäten der Mikrotitterplatten (Fall A).

Im Fall A muß gewährleistet sein, daß alle weiteren Meßparameter und sonstigen Parameter, wie beispielsweise induzierte und konstitutive Expression, bei den Organismen, die die jeweilige Lipase exprimieren, identisch sind.

Eine nach dieser Methode gemessene, größere Menge Methoxycyclohexanyllaurat pro Menge Lipase bedeutet eine höhere spezifische Aktivität.

Als enzymkatalytische Umwandlungen werden chemische Reaktionen von Substraten verstanden, die Lipasen katalysieren können. Beispielhaft seien folgende Reaktionen erwähnt:
Acylierung oder enantioselektive Acylierung von Alkoholen,
Acylierung oder enantioselektive Acylierung von Aminen,
Acylierung oder enantioselektive Acylierung von Aminoestern, wie beispielsweise Aminosäureester,
Verseifung (Hydrolyse) oder enantioselektive Verseifung (Hydrolyse) von Carbonsäureestern,
Acylierung oder enantioselektive Acylierung von Cyanhydrinen, Verseifung oder enantioselektive Verseifung von Cyanhydrinestern,
Asymmetrisierung von Meso-diolen oder
Asymmetrisierung von Meso-diestern durch Verseifung.

Bevorzugte Verfahren sind Verfahren zur Acylierung oder enantioselektive Acylierung von Alkoholen, Acylierung oder enantioselektive Acylierung von Aminen, Acylierung oder enantioselektive Acylierung von Aminoestern, wie beispielsweise Aminosäureester oder ein Verfahren zur Verseifung (Hydrolyse) oder enantioselektiven Verseifung (Hydrolyse) von Carbonsäureestern.

Das Verfahren zur enzymkatalytischen Umwandlung oder enantioselektiven Umwandlung von Substraten ist dadurch gekennzeichnet, daß man die Substrate in Gegenwart der erfindungsgemäßen Lipase-Varianten umsetzt.

Je nachdem ob es der Reaktionstyp erfordert, werden dabei vorzugsweise weitere Reagenzien zugegeben. So erfordert beispielsweise eine Acylierung die Zugabe eines Acylierungsmittels, während beispielsweise die Verseifung keine Zugabe weiterer Reagenzien benötigt.

Unter Substrat wird eine chemische Verbindung verstanden, die von Lipasen enzymkatalytisch umgesetzt, d.h. chemisch verändert werden kann. Bei enantioselektiven Umwandlungen sind Stereoisomerengemische, von denen nur ein Stereoisomeres umgesetzt wird, ebenfalls Substrate.

Beispielhaft seien Alkohole, Amine, Aminoester, Amide, Carbonsäureester, Thioester, Thiole, Cyanhydrine, Cyanhydrinester und Meso-diole und deren stereoisomerengemische als Substrate erwähnt. Bevorzugte Substrate sind Alkohole, Amine, Aminoester und Carbonsäureester bzw. racemische Alkohole, Amine, Aminoester und Carbonsäureester.

Das Verfahren wird vorzugsweise in Lösung, bei flüssigen Substraten mit oder ohne Lösungsmittel durchgeführt. Als Lösungsmittel können beispielsweise Wasser, organische Lösungsmittel oder auch wäßrig/organische Zweiphasengemische verwendet werden.

Vorzugsweise werden als organische Lösungsmittel Dioxan, THF, Diethylether, Methyl-t-butylether (MTBE), Toluol oder Heptan verwendet. Als wäßrig/organisches Zweiphasengemisch wird vorzugsweise ein Wasser/MTBE-Gemisch in beliebigem Verhältnis eingesetzt.

Bei Verfahrensdurchführung in Lösung ist die Substratkonzentration nicht kritisch, liegt aber vorzugsweise zwischen 0.5 Gew.-% und 50 Gew.-% bezogen auf die Lösung, besonders bevorzugt sind 20 bis 30 Gew.-%. Die Temperatur bei der Durchführung des Verfahrens ist ebenfalls nicht kritisch, ist aber nach oben durch die Temperaturstabilität des Enzyms beschränkt. Vorzugsweise wird das Verfahren bei 0°C bis 60°C durchgeführt, besonders bevorzugt sind 15°C bis 40°C.

Das Verfahren kann kontinuierlich oder diskontinuierlich durchgeführt werden. Für die kontinuierliche Verfahrensdurchführung leitet man beispielsweise in an sich bekannter Weise in einem Reaktor durch eine Schüttschicht aus immobilisierter Lipase-Variante eine flüssige mobile Phase. Die mobile Phase kann entweder eine Lösung von Substrat (und Reagenzien) bzw. die flüssigen Substrate (und Reagenzien) ohne Lösungsmittel sein. Die Durchflußrate ist nicht kritisch und richtet sich nach verfahrenstechnischen Gesichtspunkten wie Höhe, Durchmesser und Korngröße der Schüttschicht sowie nach der Ausgestaltung des Reaktors.

Als Reaktoren für das kontinuierliche Verfahren verwendet man vorzugsweise die für kontinuierliche, heterogenkatalytische Prozesse (Fluid/Feststoff-Reaktionen) üblichen Reaktoren (J. Hagen, Chemische Reaktionstechnik, VCH, Weinheim 1992, S. 165-169). Beispielhaft seien Wirbelschichtreaktoren und Festbettreaktoren, wie Rohrreaktor, Säulenreaktor, Vollraumreaktor, Hordenreaktor, Rohrbündelreaktor und Flachbett-Kontaktofen genannt.

In der diskontinuierlichen Verfahrensdurchführung werden die immobilisierten Lipase-Varianten in an sich bekannter Weise in einem Reaktor in einer Lösung von Substrat (und Reagenzien) bzw. in flüssigen Substraten (und Reagenzien) mit oder ohne Lösungsmittel suspendiert und die Suspension durchmischt. Als Reaktoren für das diskontinuierliche Verfahren verwendet man vorzugsweise die für diskontinuierliche, heterogenkatalytische Prozesse (Fluid/Feststoff-Reaktionen) üblichen Reaktoren mit Schüttel-, Misch- oder Rührvorrichtung. Beispielhaft sei der Rührkessel und sich davon ableitende Ausgestaltungen sowie Reaktionsgefäße mit Schüttelvorrichtung erwähnt.

Nach Beendigung der Reaktion (Erreichung des thermodynamischen Gleichgewichts) wird die immobilisierte Lipase-Variante, beispielsweise durch Dekantieren, Abzentrifugieren oder Abfiltrieren und Waschen isoliert und in weiteren Reaktionen verwendet.

In einer bevorzugten Ausführungsform des Verfahrens werden Substrate, die acylierbare funktionelle Gruppen wie z.B. Hydroxyl-oder Aminogruppen enthalten, wie Alkohole, Amine oder Aminosäureester in Gegenwart der immobilisierten Lipase als Katalysator und eines Acylierungsmittels acyliert oder enantioselektiv acyliert.

Diese enzymkatalytische Umwandlung wird bevorzugt in einem organischen Lösungsmittel wie beispielsweise Dioxan, THF, Diethylether, Methyl-t-butylether (MTBE), Toluol oder Heptan durchgeführt.

Besonders bevorzugt ist ein Verfahren zur Acylierung oder enantioselektiven Acylierung von Alkoholen, Aminen oder Aminosäureester oder racemischen Alkoholen, Aminen oder Aminosäureester in Gegenwart eines Acylierungsmittels und der Lipase-Variante der SEQ.ID.NO.1.

Hinsichtlich der Alkohole, Amine und Aminosäureester gibt es praktisch keine Beschränkung. So können ein- und mehrwertige Alkohole, wie beispielsweise
1-Phenylethanol,
gegebenenfalls substituiertes 2-Chlor-1-phenylethanol,
Pent-3-in-2-ol,
1-Butin-3-ol,
2-Hydroxy-4-phenylbuttersäureester,
a-Methyl-(1,3)-benzodioxol-5-ethanol,
2-propanol-1-(1,3-benzodioxol-4-yl),
trans-2-Methoxy-cyclohexanol,
2-Methoxy-2-phenyl-ethanol,
cis-2-Methyl-4-hydroxy-pyran oder
trans-2-Methyl-4-hydroxy-pyran
oder deren Stereoisomerengemische,
ein und mehrwertige Amine oder deren Stereoisomerengemische oder
α, β oder γ-Aminosäureester wie beispielsweise die gegebenenfalls mit Halogen substituierten C₁-C₄-Alkyl-, Alkylaryl-, Aryl-, C₂-C₆-Alkenyl- oder C₂-C₆-Alkinyl-Ester der natürlichen Aminosäuren oder deren Stereoisomerengemische
verwendet werden.

Unter gegebenenfalls substituiertem 2-Chlor-1-phenylethanol wird insbesondere 2-Chlor-1-phenylethanol und substituiertes 2-Chlor-1-phenylethanol verstanden. Bevorzugte substituierte 2-Chlor-1-phenylethanole sind Alkohole der Formel III 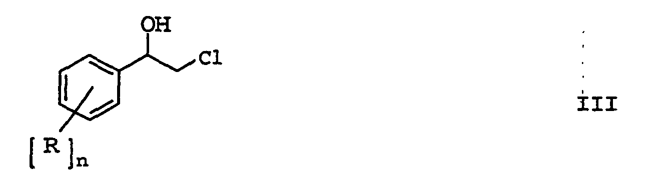 wobei
n 1 bis 3 und
R unabhängig voneinander Halogen, insbesondere F oder Cl, C₁-C₄-Alkoxy, insbesondere Methoxy und NO₂ bedeuten.

Ein besonders bevorzugtes substituiertes 2-Chlor-1-phenylethanol ist 2-Chlor-1-(m-chlorphenyl)-ethanol.

Vorzugsweise verwendet man als Substrat racemisches trans-2-Methoxy-cyclohexanol.

Als Acylierungsmittel werden organische Verbindungen verstanden, die in Gegenwart von Lipasen in Lösung als Acylüberträger fungieren können. Beispielhaft seien erwähnt:
Aliphatische, araliphatische oder aromatische Carbonsäuren die gegebenenfalls mit Halogen, wie Cl, Br, I, F substituiert sind (Acylierung), wie
   C₁-C₆-Alkancarbonsäuren, z.B. Ameisensäure, Essigsäure, Propionsäure, Buttersäure oder
   wie araliphatische oder aromatische Carbonsäuren z.B. Benzoesäure, 3-Phenylpropionsäure oder
   die entsprechenden Carbonsäureester (Umesterung) wie beispielsweise
   3-Phenylpropionsäureester oder Essigsäurealkylester, wie z.B. Essigsäureethylester.
Bevorzugte Carbonsäureester als Acylierungsmittel sind Vinylester der Formel I 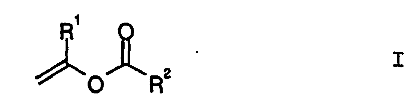 in der
   R¹ Wasserstoff oder eine C₁-C₄-Alkyl-, vorzugsweise Methylgruppe und
   R² Wasserstoff, C₁-C₁₈-Alkyl, welches gegebenenfalls mit Halogen substituiert ist, Phenyl oder (C₁-C₃-)Alkoxy-(C₁-C₄)-Alkyl bedeutet.

Bevorzugte Vinylester der Formel I sind Vinylformiat, Vinylacetat, Vinylpropionat, Vinylbutyrat oder Vinyllaurat.

Acylierungsmittel sind weiterhin aliphatische, cycloaliphatische, araliphatische oder aromatische Carbonsäureanhydride und gemischte Carbonsäureanhydride (Acylierung) wie Essigsäureanhydrid, Bernsteinsäureanhydrid (BSA), Buttersäureanhydrid, 2-Ethylhexansäureanhydrid oder Methyl-Bernsteinsäureanhydrid. Im Falle der Verwendung von Bernsteinsäureanhydrid (BSA) oder anderer schlecht löslicher Anhydride als Acylierungsmittel kann Propylencarbonat besonders vorteilhaft zugemischt werden, um das BSA in Lösung zu bringen. Dies ist vor allem im Hinblick auf ein kontinuierliches Verfahren von Bedeutung.

Ein besonders bevorzugtes Acylierungsmittel ist Vinyllaurat.

In einer weiteren bevorzugten Ausführungsform des Verfahrens werden Carbonsäureester in Gegenwart der erfindungsgemäßen Lipase-Varianten oder Lipase-Formulierungen verseift oder enantioselektiv verseift.

In diesem Fall müssen keine weiteren Reagenzien zugegeben werden, dennoch ist die Anwesenheit von Wasser nötig. Bevorzugt wir die Verseifung von Carbonsäureester durch Zusatz von Wasser unter Verwendung eines vorzugsweise zweiphasigen Systems wie z.B. Wasser/MTBE in Gegenwart der erfindungsgemäßen Lipase-Varianten oder Lipase-Formulierungen durchgeführt.

Hinsichtlich der Carbonsäureester gibt es praktisch keine Beschränkung. So können beispielsweise Verbindungen der Formel II oder deren Stereoisomerengemische 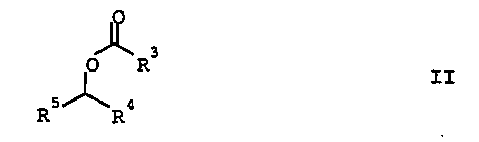 wobei
R³, R⁴ und R⁵ unabhängig voneinander Wasserstoff,
Halogen, wie beispielsweise F, Cl, Br oder I,
einen verzweigten oder unverzweigten, gegebenenfalls substituierten
C₁-C₈-Alkylrest wie beispielsweise gegebenenfalls substituiertes Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl 1-Ethyl-2-methylpropyl, Heptyl oder Octyl,
C₂-C₆-Alkenylrest, wie beispielsweise gegebenenfalls substituiertes 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl oder 1-Ethyl-2-methyl-2-propenyl,
C₃-C₆-Alkinylrest wie beispielsweise gegebenenfalls substituiertes 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-1-methyl-2-propinyl
oder C₃-C₈-Cycloalkylrest, wie beispielsweise gegebenenfalls substituiertes Cyclopropyl, Cyclobutyl, Cyclopentyl, Cyclohexyl und Cycloheptyl, Cyclooctyl,
einen gegebenenfalls substituierten
Arylrest, wie beispielsweise gegebenenfalls substituiertes Phenyl, 1-Naphtyl oder 2-Naphtyl,
Arylalkylrest, wie beispielsweise gegebenenfalls substituiertes Benzyl,
Heteroarylrest, wie beispielsweise gegebenenfalls substituiertes 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl oder 6-Pyridazinyl, vorzugsweise 2-Pyridyl, 3-Pyridyl, 4-Pyridyl, 2-Furyl, 3-Furyl, 2-Thienyl, 3-Thienyl, 2-Thiazolyl, 4-Thiazolyl oder 5-Thiazolyl,
oder Heterocycloalkyl- oder -alkenylrest bedeuten.

Als ein bis dreifache Substituenten der C₁-C₈-Alkyl-, C₂-C₆-Alkenyl-, C₂-C₆-Alkinyl-, C₃-C₈-Cycloalkyl-, Aryl-, Arylalkyl-, Heteroaryl-, Heterocycloalkyl- oder -alkenylreste kommen beispielsweise Halogen-, Nitro-, Amino-, Hydroxy- oder Cyanogruppen, C₁-C₄-Alkyl-, C₁-C₄-Halogenalkyl-, C₁-C₄-Alkoxy-, C₁-C₄-Halogen-alkoxy-, C₁-C₄-Alkylthio-, Hetaryl-, Arylreste oder der Rest -O-CO-C₁-C₄-Alkyl
in Frage.

Bevorzugte Carbonsäureester sind beispielsweise
1-Butin-3-ol-acetat,
1-Butin-3-ol-butyrat,
Essigsäure-1-phenyl-ethylester oder
2-Acetoxy-4-phenylbuttersäureester.

Das Verfahren zur enantioselektiven enzymkatalytischen Umwandlung von Substraten mit den erfindungsgemäßen Lipase-Varianten oder Lipase-Formulierungen kann zur Abtrennung von Stereoisomeren und insbesondere zur Abtrennung von Enantiomeren oder Diastereomeren aus einem Stereoisomerengemisch des Substrats dienen. Besonders bevorzugt dient es zur Abtrennung von Enantiomeren oder Diastereomeren aus racemischen Substraten und damit zur Herstellung von optisch aktiven Verbindungen aus den jeweiligen racemischen Gemischen.

Durch die enantioselektive Substratspezifität der erfindungsgemäßen Lipase-Varianten oder Lipase-Formulierungen, wird beispielsweise nur ein Enantiomeres des racemischen Substrats umgewandelt, das andere Enantiomere reagiert nicht. Die entstehenden Produkte lassen sich durch chemische, physikalische und mechanische Trennmethoden in an sich bekannter Weise leicht trennen. Beispielhaft seien Kristallisation, Fällung, Extraktion in zweiphasigen Lösungsmittelsystemen, chromatographische Trennverfahren, wie HPLC, GC oder Säulenchromatographie über Kieselgel oder thermische Trennungsverfahren wie Destillation genannt.

Demgemäß betrifft die vorliegende Erfindung weiterhin ein Verfahren zur Herstellung optisch aktiver Verbindungen, dadurch gekennzeichnet, daß man Stereoisomerengemische oder Racemate von Subtraten, die sich enzymkatalytisch von Lipasen umsetzen lassen, enantioselektiv in Gegenwart der erfindungsgenmäßen Lipase-Varianten umsetzt und anschließend die Gemische auftrennt.

Mit dem erfindungsgemäßen Verfahren lassen sich vorzugsweise die optisch aktiven Verbindungen herstellen, die als Stereoisomerengemisch als Substrate von Lipasen umgesetzt werden können, bzw. von deren Stereoisomerengemisch mindestens ein Stereoisomer als Substrat von Lipasen umgesetzt werden kann.

Ein Verfahren zur enantioselektiven Acylierung von Alkoholen, Aminen oder Aminosäureester dient vorzugsweise zur Trennung von racemischen Alkoholen, Aminen bzw. Aminosäureester und damit zur Herstellung von optisch aktiven Alkoholen, Aminen bzw. Aminosäureester.

Ein Verfahren zur enantioselektiven Hydrolyse oder Verseifung von Carbonsäureestern dient vorzugsweise zur Trennung von racemischen Carbonsäureestern und damit zur Herstellung von optisch aktiven Carbonsäureestern.

Die erfindungsgemäßen Lipase-Varianten oder Lipase-Formulierungen weisen den Vorteil auf, daß sie bei gleichbleibender Enantioselektivität eine erhöhte spezifische Aktivität aufweisen.

Die nachstehenden Beispiele erläutern die Erfindung:
Allgemeine experimentelle Bedingungen

In allen Fällen, in denen keine detaillierte Beschreibung der Experimente erfolgt, wurden molekularbiologische Methoden angewandt, die bekannt sind und z.B. in Current Protocols of Molecular Biology, 1999, John Wiley & Sons beschrieben sind.

Verwendete Bakterienstämme:
Escherichia coli XL1-Blue (Fa. Stratagene)
Escherichia coli XJS5037 (pRK2013) (Proc. Natl. Acad. Sci., 1979, 1648-1652)
Pseudomonas spec. DSM 8246

Verwendete Plasmide:
pBluescriptIIKS (Fa. Stratagene),
pBP1500: Abkömmling von pML131 (Gene, 1990, 89:37-46) mit einem 2,6 kBp-DNA-Fragment, welches das Lipase-Operon aus *Pseudomonas spec. DSM 8246* enthält. pBP1500 codiert für eine enzymatisch inaktive Lipase (lipA⁻),
pBP1520: Abkömmling von pML131 (Gene, 1990, 89:37-46) mit einem 2,6 kBp-DNA-Fragment, welches das Lipase-Operon aus *Pseudomonas spec. DSM 8246* enthält. pBP1520 codiert für eine enzymatisch aktive Lipase (lipA),
pBP2112: Abkömmling von pML131 (Gene, 1990, 89:37-46) mit einem 2,6 kBp-DNA-Fragment, elches das Lipase-Operon aus *Pseudomonas spec. DSM 8246* enthält und das die Mutation zur Produktion der Lipasevariante LipA L17A enthält.

### Beispiel 1

Herstellung einer Lipase-Variante mit gesteigerter spezifischer Enzymaktivität
A) Kultivierung der Bakterien
   Die rekombinanten Stämme von Escherichia coli wurden entweder auf festem LB-Agar kultiviert oder in LB-Medium 16 h im Rundschüttler mit 200 Upm bei 37°C. Das Medium enthielt die zur Elektion erforderlichen Antibiotika *Pseudomonas spec. DSM 8246* und die rekombinanten Abkömmlinge dieses Stammes wurden entweder auf festem FG-Agar oder in FP-Medium 24 h im Rundschüttler mit 200 Upm bei 30°C oder in (NH₄)₂SO₄ 6 g/l, CaCl₂ 0,02 g/l, MgSO₄ x 7 H₂I 1 g/l, KH₂PO₄ 3,5 g/l, K₂HPO₄ 3,5 g/l, Hefeextrakt 5 g/l, Glucose 5 g/l, Spurensalzlösung 10 ml/l 24 h im Rundschüttler bei 150 Upm bei 30°C inkubiert. Das Medium erhielt die zur Selektion erforderlichen Antibiotika. Als Inkubationsgefäße wurden Petrischlan, Kulturröhrchen oder Mikrotiterplatten verwendet.
   Folgende Antibiotika (Selektion) wurden verwendet: Tetracyclin 10 µg/ml ( XL1-Blue), Ampicillin 100 µg/ml (pBluescriptIIKS), Gentamycin 10 µg/ml (pBP-Plasmide in E. coli), Gentamycin 45 µg/ml (pBP-Plasmide in *Pseudomonas spec. DSM 8246*), Chloramphenicol 20 µg/ml (*Pseudomonas spec. DSM 8246*), Kanamycin 25 µg/ml (Escherichia coli XJS5037 (pRK2013) oder *Pseudomonas spec. DSM 8246*)
B) Mutagenese
   Durch ortsgerichtete Mutagenese mit der Methode Overlap-Extension-PCR (Recombinant PCR, 1990, 177-183, Academic Press, San Diego) wurde die Mutante L17A von *lipA* aus *Pseudomonas spec. DSM 8246* erzeugt.
   Chromosomale DNA von *Pseudomonas spec. DSM 8246* diente als Matrizen-DNA. Als Primer wurden eingesetzt:
   MAT16 5'-GATCGACGTAAGCTTTAACGATGGAGAT-3' (SEQ.ID.NO.6)
   MAT109 5'-CGGTGCCCGCGGCGCCGTGGACGAGGATCACCG-3' (SEQ.ID.NO.3)
   MAT108 5'-CGTCCACGGCGCCGCGGGCACCGACAAGTTCGC-3' (SEQ.ID.NO.4)
   MAT19 5'-CATCGGGCGAGCTCCCAGCCCGCCGCG-3' (SEQ.ID.NO.5)
   Im ersten PCR-Schritt wurde mit MAT16 und MAT109 ein Teilfragment von *lipA* und mit MAT108 und MAT19 ein weiteres Teilfragment von *lipA* erzeugt. Die Teilfragmente wurden im Agarosegel aufgetrennt und mit dem GFX Kit (Fa. Pharmacia) gereinigt. Aus den gereinigten Fragmenten wurde im zweiten PCR-Schritt mit MAT16 und MAT19 das Fragment *lipA** erzeugt, das für die Lipasevariante L17A codiert.
   Die PCR wurde nach der Vorschrift des Herstellers mit dem GC-Rich Kit (Fa. Pharmacia) mit dem folgenden Temperaturprofil durchgeführt: (1) 95°C - 3 min, (2) 95°C - 30 s, (3) 40°C - 30 s, (4) 72 - 30 s, (5) 95°C - 30 s, (6) 60°C - 30 s, (7) 72°C - 30 s, (8) 72°C - 10 min; Schritte (2) - (4) mit 5 Zyklen; dann Schritte (5) - (7) 15 Zyklen.
   Das Fragment *lipA** und der Vektor pBluescriptIIKS wurden mit HindIII und *Sac*I gespalten, im Agarosegel getrennt, mit dem GFX Kit gereinigt und ligiert. Anschließend wurde der Ansatz in E. coli transformiert. Das rekombinante Plasmid wurde gereinigt und sequenziert, um die Mutation nachzuweisen. Bis auf die Mutation L17A wurden keine weiteren Mutationen in der DNA nachgewiesen.
C) Klonierung
   Der Vektor pBP1500 und das rekombinante pBluescript-Derivat mit *lipA** wurde mit HindIII und *Sac*I gespalten und ligiert. Das entstandene rekombinante Plasmid wurde als pBP2112 bezeichnet. pBP2112 wurde in *E. coli* XL1-Blue transformiert. Anschließend wurde pBP2112 durch Konjugation von E. coli XL1-BLue mit Hilfe des Helferstammes *E. coli* XJS5037 (pRK2013) nach *Pseudomonas spec. DSM 8246* übertragen. Der rekombinante Stamm wurde als *Pseudomonas spec. DSM 8246* (pBP2112) bezeichnet.
D) Enzymproduktion und Aufarbeitung
   Pseudomonas spec. DSM 8246 (pBP2112) wurde in 96-Kavitäten-Mikrotiterplatten mit je 250 µl Medium pro Kavität auf dem Rundschüttler mit 150 Upm bei 30°C für 24 h inkubiert. Als Kontrolle wurde Pseudomonas spec. DSM 8246 (pBP1520) angezogen. Dieser Stamm trägt plasmidcodiert das lipA-Gen von Pseudomonas spec. DSM 8246 (Vergleichsbeispiel). Für die Anzucht der Bakterien wurde folgendes Medium eingesetzt: (NH₄)₂SO₄ 6g/l, CaCl₂ 0,02g/l, MgSO₄ x 7 H₂O 1g/l, KH₂PO₄ 3,5g/l, K₂HPO₄ 3,5g/l, Hefeextrakt 5g/l, Glucose 5g/l, Spurensalzlösung 10 ml/l, Kanamycin 25 µg/ml, Gentamycin 45µg/ml. Nach der Anzucht wurden die Mikrotiterplatten zentrifugiert. Die Kulturüberstände wurden anschließend in eine Polypropylen-Mikrotiterplatte übertragen, 16 h bei -80°C gefroren und dann 48 h gefriergetrocknet.
   Unter den Kultivierungsbedingungen wird die chromosomale Kopie des Lipasegens nicht exprimiert. Daher trägt auch das Vergleichsbeispiel die natürliche Lipase plasmidcodiert.
E) Racematspaltung und Analytik

In den Mikrotiterplatten mit den gefriergetrockneten Überständen wurde die Racematspaltung von rac-trans-2-Methoxycyclohexanol durchgeführt. Für die Racemattrennung wurden Methoxycyclohexanol und Vinyllaurat im molaren Verhältnis 1 : 0,65 eingesetzt [d.h. pro Platte 10 g Methoxycyclohexanol und 11,3 g Vinyllaurat]. In jede Kavität wurden 200 µl dieses Gemisches pipettiert. Jede Platte wurden mit 'plate sealer' versiegelt, mit Deckel verschlossen und 96 h bei Raumtemperatur im Rundschüttler bei 150 U/min inkubiert.

Anschließend wurden jeweils 100 Mikroliter pro Kavität abgenommen mit Essigsäureethylester versetzt und gaschromatographisch analysiert.

Tabelle 1 enthält die gemessene Menge gebildetes Methoxycyclohexanyllaurat in Gew.%.

**Tabelle 1**

| Beispiel 1E Menge gebildetes Methoxycyclohexanyllaurat in [Gew.%] unter Verwendung der Lipase-Variante LipA L17A aus *Pseudomonas spec. DSM 8246* (pBP2112) | Vergleichsbeispiel Menge gebildetes Methoxycyclohexanyllaurat im [Gew.%] unter Verwendung der Lipase aus *Pseudomonas spec. DSM 8246* (pBP1520) | Medienleerwert |
|---|---|---|
| 14,9 | 2,6 | 0,02 |
| 18,6 | 1,0 | 0,02 |
| 21,2 | 2,5 | 0,02 |
| 18,1 | 2,7 | 0,02 |
| 16,8 | 2,2 | 0,03 |
| 14,0 | 1,7 | 0,02 |
| 15,1 | 1,8 | 0,03 |
| 10,3 | 0,8 | 0,02 |
| Mittel = 16,1 | Mittel = 1,9 | Mittel = 0,02 |

Die Lipasevariante LipA L17A zeigte im Vergleich zum natürlichen Enzym eine um den Faktor 8,5 höhere Aktivität in der Racematspaltung von Methoxycyclohexanol.

Die spezifische Enzymaktivität ist in diesem Fall definiert als Menge gebildetes Methoxycyclohexanyllaurat in [Gew.%] geteilt durch Optische Dichte der Bakteriensuspension bei OD₆₆₀ₙₘ. Die gebildete Menge aktives Enzym korreliert im Bereich einer optischen Dichte von OD^{660nm} = 0,7 +/- 0,2 linear mit der Trübung der Bakteriensuspension.

Dabei ergaben sich für die spezifische Aktivität folgende Werte:

**Tabelle 2**

| Spezifische Aktivität der Lipase-Variante LipA L17A aus *Pseudomonas spec. DSM 8246* (pBP2112) in der Reaktion nach Beispiel 1 E in [Gew.-%] | Vergleichsbeispiel: Spezifische Aktivität der Lipase aus *Pseudomonas spec. DSM 8246* (pBP1520) in der Reaktion nach Beispiel 1 E in [Gew.-%] | Medienleerwert |
|---|---|---|
| 16,4 | 3,1 | 0,5 |
| 20,2 | 1,2 | 0,5 |
| 22,1 | 3,0 | 0,5 |
| 20,3 | 3,4 | 0,5 |
| 19,1 | 2,8 | 0,8 |
| 14,4 | 2,2 | 0,5 |
| 17,4 | 2,4 | 0,8 |
| 10,8 | 1,0 | 0,5 |
| Mittel =17,6 | Mittel = 2,4 | Mittel = 0,6 |

Die Lipasevariante LipA L17A zeigte im Vergleich zum natürlichen Enzym eine um den Faktor 7,3 höhere spezifiche Enzymaktivität in der Racematspaltung von Methoxycyclohexanol.

### SEQUENCE LISTING

<160> 1
<170> PatentIn Ver. 2.0
<210> 1
   <211> 319
   <212> PRT
   <213> Pseudomondadaceae gen. sp.
<400> 1
   Ala Asp Thr Tyr Ala Ala Thr Arg Tyr Pro Val Ile Leu Val His Gly
    1               5                  10                  15
   Leu Ala Gly Thr Asp Lys Phe Ala Asn Val Val Asp Tyr Trp Tyr Gly
               20                  25                  30
   Ile Gln Ser Asp Leu Gln Ser His Gly Ala Lys Val Tyr Val Ala Asn
           35                  40                  45
   Leu Ser Gly Phe Gln Ser Asp Asp Gly Pro Asn Gly Arg Gly Glu Gln
       50                  55                  60
   Leu Leu Ala Tyr Val Lys Gln Val Leu Ala Ala Thr Gly Ala Thr Lys
   65                  70                  75                  80
   Val Asn Leu Ile Gly His Ser Gln Gly Gly Leu Thr Ser Arg Tyr Val
                   85                  90                  95
   Ala Ala Val Ala Pro Gln Leu Val Ala Ser Val Thr Thr Ile Gly Thr
               100                 105                 110
   Pro His Arg Gly Ser Glu Phe Ala Asp Phe Val Gln Asp Val Leu Lys
           115                 120                 125
   Thr Asp Pro Thr Gly Leu Ser Ser Thr Val Ile Ala Ala Phe Val Asn
       130                 135                 140
   Val Phe Gly Thr Leu Val Ser Ser Ser His Asn Thr Asp Gln Asp Ala
   145                 150                 155                 160
   Leu Ala Ala Leu Arg Thr Leu Thr Thr Ala Gln Thr Ala Thr Tyr Asn
                   165                 170                 175
   Arg Asn Phe Pro Ser Ala Gly Leu Gly Ala Pro Gly Ser Cys Gln Thr
               180                 185                 190
   Gly Ala Ala Thr Glu Thr Val Gly Gly Ser Gln His Leu Leu Tyr Ser
           195                 200                 205
   Trp Gly Gly Thr Ala Ile Gln Pro Thr Ser Thr Val Leu Gly Val Thr
       210                 215                 220
   Gly Ala Thr Asp Thr Ser Thr Gly Thr Leu Asp Val Ala Asn Val Thr
   225                 230                 235                 240
   Asp Pro Ser Thr Leu Ala Leu Leu Ala Thr Gly Ala Val Met Ile Asn
                       245                           250                           255
   Arg Ala Ser Gly Gln Asn Asp Gly Leu Val Ser Arg Cys Ser Ser Leu
                   260                           265                           270
   Phe Gly Gln Val Ile Ser Thr Ser Tyr His Trp Asn His Leu Asp Glu
             275                           280                           285
   Ile Asn Gln Leu Leu Gly Val Arg Gly Ala Asn Ala Glu Asp Pro Val
        290                           295                           300
   Ala Val Ile Arg Thr His Val Asn Arg Leu Lys Leu Gln Gly Val
   305                           310                           315

## Patentansprüche

1. Lipase-Variante, herstellbar, indem man an der Aminosäuresequenz einer Ausgangslipase SEQ ID No 1 eine Aminosäuresubstitution an Position 17 durchführt, wobei Leu¹⁷ ersetzt wird durch Ala¹⁷.

2. Nukleinsäuresequenz, kodierend eine Lipase-Variante gemäß Anspruch 1.

3. Nicht-menschlicher Wirtsorganismus der mit einer Nukleinsäuresequenz gemäß Anspruch 2 transformiert worden ist.

4. Verfahren zur Herstellung einer Lipase-Variante gemäß Anspruch 1, wobei ein Wirtsorganismus gemäß Anspruch 3 unter Bedingungen kultiviert wird, die die Produktion der Lipase-Variante erlauben und die Lipase-Variante anschließend aus dem Kulturmedium gewonnen wird.

5. Verfahren zur enzymkatatytischen Umwandlung oder enantioselektiven Umwandlung von Substraten, **dadurch gekennzeichnet, daß** man die Substrate in Gegenwart der Lipase-Varianten gemäß Anspruch 1 umsetzt.

6. Verfahren nach Anspruch 5, **dadurch gekennzeichnet, daß** man als Substrate Alkohole, Amine oder Aminosäureester verwendet und diese in Gegenwart eines Acylierungsmittels acyliert.

7. Verfahren zur Herstellung optisch aktiver Verbindungen, **dadurch gekennzeichnet, daß** man Stereoisomerengemische oder Racemate von Substraten, die sich enzymkatalytisch von Lipasen umsetzen lassen, enantioselektiv in Gegenwart der Lipase-Varianten gemäß Anspruch 1 umsetzt und anschließend die Gemische auftrennt.

8. Verfahren nach Anspruch 7, **dadurch gekennzeichnet, daß** man als Substrate Alkohole, Amine oder Aminosäureester verwendet und diese in Gegenwart eines Acylierungsmittels enantioselektiv acyliert,

9. Verwendung der Lipase-Varianten gemäß Anspruch 1 als Katalysator.

## Claims

1. A lipase variant obtainable by carrying out, on the amino acid sequence of a starting lipase SEQ ID No 1, an amino acid substitution in position 17. Leu¹⁷ being replaced by Ala¹⁷.

2. A nucleic acid sequence encoding a lipase variant according to claim 1.

3. A non-human host organism which has been transformed using a nucleic acid sequence according to claim 2.

4. A process for the preparation of a lipase variant according to claim 1, wherein a host organism according to claim 3 is cultured under conditions which the production of the lipase variant and the lipase variant subsequently is recovered from the culture medium.

5. A process for the enzme-catalytic conversion or enantioselective conversion of substrates, which comprises reacting the substrates in the presence of the lipase variants according to claim 1.

6. The process according to claim 5, wherein the substrates used are alcohols, amines or amino acid eaters and these are acylated in the presence of an acylating agent.

7. A process for the preparation of optically active compounds, which comprises reacting stereoisomer mixtures or racemates of substrates which can be reached in an enzyme-catalyzed manner by lipases enantioselectively in the presence of the lipase variants according to claim 1 and then resolving the mixtures.

8. The process according to claim 7, wherein the substrates used are alcohols, amines or amino acid esters and these are enantioselectively acylated in the presence of an acylating agent.

9. The use of the lipase variants according to claim 1 as catalyst.

## Revendications

1. Variante de lipase obtenue en procédant, au niveau de la séquence d'acides aminés d'une lipase de départ de séquence SEQ ID n° 1, à une substitution d'acides aminés au niveau de la position 17, Leu¹⁷ étant remplacé par Ala¹⁷.

2. Séquence d'acides nucléiques codant une variante de lipase selon la revendication 1.

3. Organisme hôte non humain qui a été transformé avec une séquence d'acides nucléiques selon la revendication 2.

4. Procédé servant à fabriquer une variante de lipase selon la revendication 1, un organisme hôte selon la revendication 3 étant cultivé dans des conditions qui autorisent la production de la variante de lipase, et la variante de lipase étant obtenue par la suite à partir du milieu de culture.

5. Procédé servant à convertir des substrats à l'aide d'une catalyse enzymatique ou de manière énantiosélective, **caractérisé en ce que** l'on convertit les substrats en présence des variantes de lipase selon la revendication 1.

6. Procédé selon la revendication 5, **caractérisé en ce que** des alcools, des amines ou des esters d'acides aminés sont utilisés comme substrats et qu'ils sont acylés en présence d'un agent d'acylation.

7. Procédé servant à fabriquer des composés optiquement actifs, **caractérisé en ce que** des mélanges de stéréoisomères ou des composés racémiques de substrats, lesquels sont convertibles par des lipases de manière catalysée par enzyme, sont convertis de manière énantiosélective en présence des variantes de lipase selon la revendication 1, puis les mélanges sont séparés.

8. Procédé selon la revendication 7, **caractérisé en ce que** des alcools, des amines ou des esters d'acides aminés sont utilisés comme substrats et qu'ils sont acylés en présence d'un agent d'acylation de manière énantiosélective.

9. Utilisation des variantes de lipase selon la revendication 1 comme catalyseur.
